# EUROPEAN PATENT SPECIFICATION

(11) **EP 3 022 297 B1**
(45) Date of publication and mention of the grant of the patent: **05.07.2023**
(21) Application number: 14747317.7
(22) Date of filing: 16.07.2014
(51) Int. Cl.: C12N 7/00, A61K 35/76

(54) **GENETICALLY STABLE ONCOLYTIC RNA VIRUS, METHOD OF MANUFACTURING AND USE THEREOF**
GENETISCH STABILES ONKOLYTISCHES RNA-VIRUS, VERFAHREN ZUR HERSTELLUNG UND VERWENDUNG DAVON
VIRUS D'ARN ONCOLYTIQUE GÉNÉTIQUEMENT STABLE, SON PROCÉDÉ DE FABRICATION ET UTILISATION ASSOCIÉE

(30) Priority: 16.07.2013 EP 13176757
(43) Date of publication of application: 25.05.2016
(73) Proprietor: Ditesan Ltd., Riga 1026 (LV)
(72) Inventor: VENSKUS, Dite, LV-3008 Jelgava (LV); KALVINS, Ivars, LV-1006 Riga (LV); PJANOVA, Dace, LV-1057 Riga (LV); PETROVSKA, Ramona, LV-1083 Riga (LV); AUZINS, Jurgis, LV-2114 Olaine (LV)
(74) Representative: Schröer, Gernot H.
(86) International application number: PCT/EP2014/065277
(87) International publication number: WO 2015/007788

(56) References cited:
- EP-A1- 1 537 872
- FERDAT A K: "MECHANISM OF IMMUNOMODULATION IN THE ANTI-TUMOUR EFFECT OF THE ECHO-7 ENTEROVIRUS", EKSPERIMENTAL NAA ONKOLOGIA - EXPERIMENTAL ONCOLOGY, KIEV, UU, vol. 11, no. 5, 1 January 1989 (1989-01-01), pages 43-48, XP008054566, ISSN: 0204-3564
- CHUA B H ET AL: "Comparison of the complete nucleotide sequences of echovirus 7 strain UMMC and the prototype (Wallace) strain demonstrates significant genetic drift over time", November 2001 (2001-11), JOURNAL OF GENERAL VIROLOGY, VOL. 82, NR. 11, PAGE(S) 2629-2639, XP002717948, ISSN: 0022-1317 page 2629

## Description

### TECHNICAL FIELD

The invention relates to development of a novel biotechnologically produced anti-cancer preparation, namely to a genetically stable oncolytic RNA virus, a method for manufacturing the oncolytic virus, and use thereof.

### BACKGROUND ART

The ability of viruses to kill cancer cells is known for more than a century [Kelly, E.; Russell, S.J. History of oncolytic viruses: genesis to genetic engineering. Mol. Ther. 2007, 15, pp. 651-659] and there were numerous promising successes in experimental cancer therapy with various viruses, nevertheless their use in clinical practice is hampered by the difficulty to foresee the interaction between the tumour and its host, as well as the virus and response of human immune system to viral antibodies.

Although the clinical investigations regarding the use of viruses in cancer therapy commenced more than 50 years ago, at present only two viruses are approved for clinical use in cancer therapy. They are adenovirus with deleted E1 B 55K gene (Garber, K. China approves world's first oncolytic virus therapy for cancer treatment. J. Natl. Cancer Inst. 2006, 98, pp. 298-300) and unmodified passivized Picornaviridae Enterovirus of Echo type (Eurasian Patent 007839; and European Patent 1537872 - European Patent Application Number EPA 03733607), acting as antitumour immunostimulant. EP 1537872 discloses the use of an ECHO-7 virus in the active immunotherapy of tumours. Virus was recovered from the intestinal tract, and isolated in a human primary embryonic cell culture. Page 2, paragraph 18 of the A1 printed specification states that nonpathogenic serotypes of virus (in particular ECHO-7), were selected. Virus was cultured in tumour tissue, then in human embryonic fibroblasts, and the process repeated. The virus obtained was demonstrated as being able to improve survival in patients with skin melanomas.

The development of novel efficient oncolytic viruses is still a topical problem (Han Hsi Wong, Nicholas R. Lemoine,Yaohe Wang, Oncolytic Viruses for Cancer Therapy: Overcoming the Obstacles, Viruses 2010, 2, pp. 78-106).

In order to increase the potential of virus selectively to infect cancer cells and heighten the oncolytic activity, a number of modified viruses have been disclosed. They are characterised by deletion of specific genes, thus preventing their propagation in normal cells, or integration of additional genes for improving the oncolytic properties.

However, the limited knowledge concerning the genetical modifications that provide for selectivity and efficiency against the tumour cells, results in modified viruses with lower cytolytic activity, compared to origin, or higher anti-virus response of human immune system (S. Meerani, Yang Yao, Oncolytic viruses in cancer therapy. European Journal of Scientific Research, vol. 40 no.1 (2010), pp.156-171 ; Han Hsi Wong, Nicholas R. Lemoine, Yaohe Wang, Oncolytic Viruses for Cancer Therapy: Overcoming the Obstacles, Viruses 2010, 2, 78-106). Although viruses are well-established tools for conveying vectors into cell, their use is limited by the high immunogenicity of viruses (Peng, Z. Current status of gendicine in China: recombinant human Ad-p53 agent for treatment of cancers. Hum. Gene. Ther. 2005, 16, 1016-1027).

One of the most serious adverse properties of non-modified ECHO type viruses, including ECHO 7, is their ability to cause infections that may have a fatal result (Wreghitt T.G., Gandy G.M., King A., Sutehall G., Fatal neonatal ECHO 7 virus infection, The Lancet, vol. 324, p.465, 1984). These viruses are known to be responsible for hand, foot and mouth disease in Malaysia
(http: / / www.vadscorner.com / echovirus7.html), for myocarditis in leukemic child (Midula M., Marzetti G., Borra G., Sabatino G., Myocarditis associated with ECHO 7 type infection in leukemic child, Acta Paediatrica Volume 65, Issue 4, pp. 649-651 , July 1976), aseptic meningitis, paralytic disease and fever (https: 11 web. archive. org / web / 20120805213326 / http: / / virology-online.com / viruses/ enteroviruses6.htm). Therefore pathogenicity is one of the major limitations that must be overcome in using ECHO 7 type viruses in treating cancer patients.

### DISCLOSURE OF THE INVENTION

### Technical problem

Therefore, the problem to solve was the development a highly efficient, selective oncolytic virus without pathogenicity in normal cells and low immunological response, and possessing high genetic stability. It is well known and recognised that RNA viruses mutate very easily upon passage in cell cultures, which can change the phenotype, leading to increased pathogenicity. Thus, for preparation of oncolytic virus-based medicine by using a wild non-pathogenic ECHO 7 virus strain as the starting material, it is of extreme importance to find a procedure which would allow to generate an oncolytic modification of this virus that would retain non-pathogenic character of the original virus and be genetically stable.

### Solution to the problem

This problem was surprisingly solved by a targeted modification of a singlestrand RNA virus by developing a method that utilizes the high mutation potential of single strand RNA virus in combination with a specifically targeted selection of mutants, providing for fast separation from the pool of mutant species with high and selective oncolytic activity. Many cancer cells are resistant to the virus (the virus can not enter the cell and survive there). By careful selection of cell lines where the virus is modified and by proper pretreatment of the cancer cells it is possible to create a genetically stable and non-pathogenic virus for cancer treatment. The virus provided by the invention is in fact the first disclosure of a genetically stable oncolytic virus, based on ECHO-7 type virus, said genetically stable virus bring usable for long term manufacturing (a multiple reproduction) as medicine.

### SHORT DESCRIPTION OF THE INVENTION

We have developed a method for modifying the native ECHO 7 virus, identified by genome sequence SeqNo2, the method comprising initially conducting the virus adaptation in cancer cells, attenuated by an anti-cancer agent such as dacarbazine, passaging the modified virus in human embryonal fibroblast culture, propagation in human melanoma cells and passaging in human embryonal fibroblast culture, optionally treated by ribavirin, isolation of the virus and purification of the virus. The virus can be isolated and purified by known methods. The use of anticancer agent such as dacarbazine in subtoxic concentrations for modification of cancer cells and using these treated cancer cells as host cells for virus replication has led to the creation of a mutant virus with a stable genome applicable as a highly effective medicine for the treatment of cancer.

The virus adaptation can be conducted in more than one type of cell line.

### BRIEF DESCRIPTION OF THE FIGURES

Fig. 1 is a comparison of genomes of the modified virus (Seq ID No 1) and unmodified (native) virus (Seq ID No 2), and

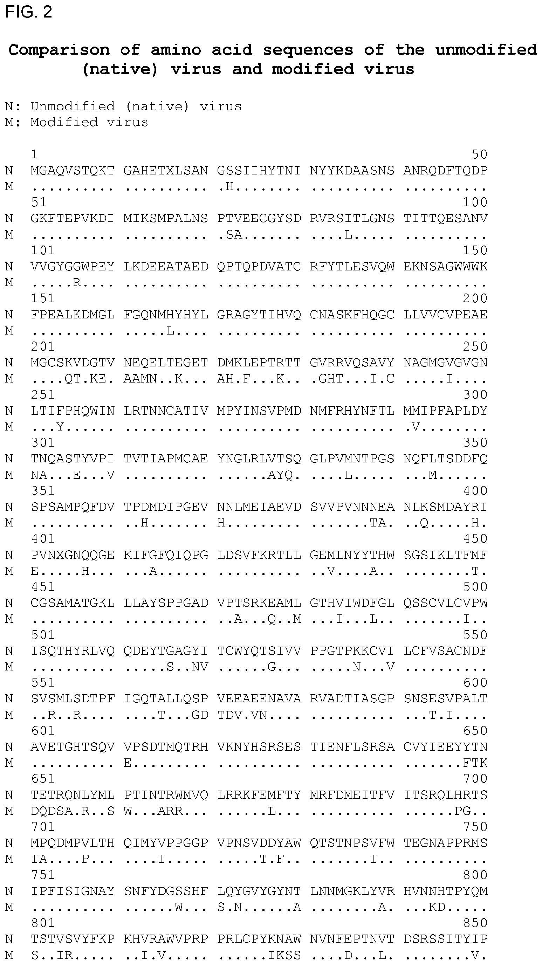

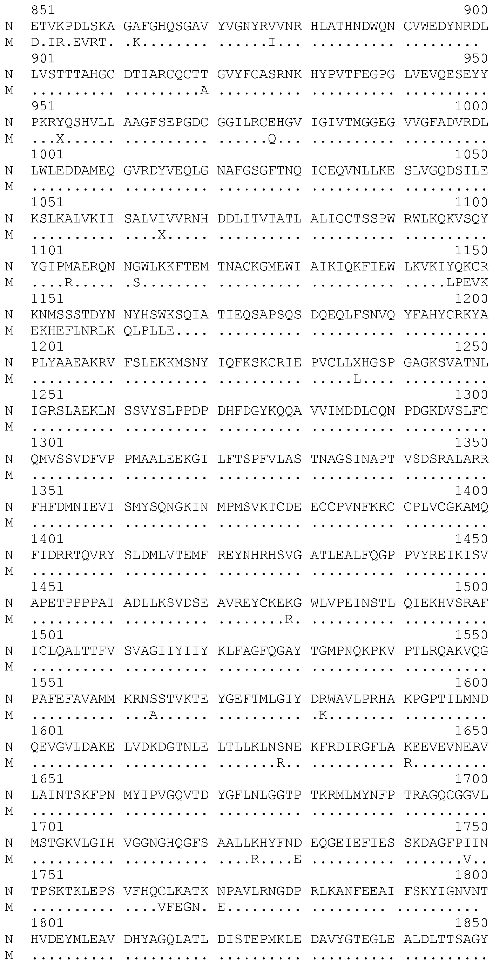

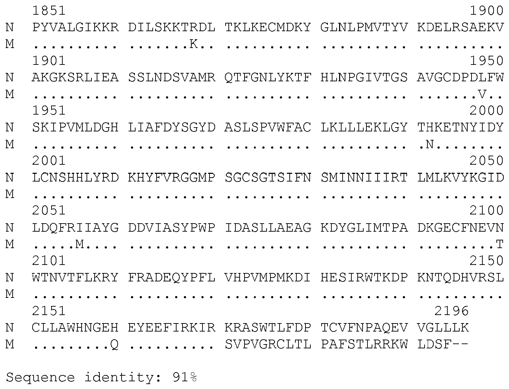

Fig. 2 is a comparison of amino acid sequences of the modified virus (Seq ID No 4) and unmodified (native) virus (Seq ID No 5).

### SEQUENCE LISTING FREE TEXT

Seq ID No 1: Modified virus;
Seq ID No 2: Unmodified (native) virus;
Seq ID No 3: Modified virus after propagation for 12 months;
Seq ID No 4: Amino acid sequence of the modified virus;
Seq ID No 5: Amino acid sequence of the unmodified virus;
Seq ID No 6: Primer Eo7-1F; Seq ID No 7: Primer Eo7-1R;
Seq ID No 8: Primer Eo7-2F; Seq ID No 9: Primer Eo7-2R;
Seq ID No 10: Primer Eo7-3F; Seq ID No 11 : Primer Eo7-3R;
Seq ID No 12: Primer Eo7-4F; Seq ID No 13: Primer Eo7-4R;
Seq ID No 14: Primer Eo7-5F; Seq ID No 15: Primer Eo7-5R;
Seq ID No 16: Primer Eo7-6F; Seq ID No 17: Primer Eo7-6R;
Seq ID No 18: Primer Eo7-7F; Seq ID No 19: Primer Eo7-7R;
Seq ID No 20: Primer Eo7-8F; Seq ID No 21: Primer Eo7-8R;
Seq ID No 22: Primer Eo7-9F; Seq ID No 23: Primer Eo7-10F;
Seq ID No 24: Primer Eo7-9R; Seq ID No 25: Primer Eo7-11F;
Seq ID No 26: Primer Eo7-11R; Seq ID No 27: Primer Eo7-12F;
Seq ID No 28: Primer Eo7-12R; Seq ID No 29: Primer Eo7-13F;
Seq ID No 30: Primer Eo7-13R; Seq ID No 31: Primer Eo7-14F;
Seq ID No 32: Primer Eo7-14R; Seq ID No 33: Primer Eo7-15F;
Seq ID No 34: Primer Eo7-15R; Seq ID No 35: Primer Eo7-16F;
Seq ID No 36: Primer Eo7-17F; Seq ID No 37: Primer Eo7-16R;
Seq ID No 38: Primer Eo7-18F; Seq ID No 39: Primer Eo7-18R;
Seq ID No 40: Primer Eo7-19F; Seq ID No 41: Primer Eo7-19R;
Seq ID No 42: Primer Eo7-20F; Seq ID No 43: Primer Eo7-20R;
Seq ID No 44: Primer Eo7-21F; Seq ID No 45: Primer Eo7-21R;
Seq ID No 46: Primer Eo7-22F; Seq ID No 47: Primer Eo7-22R;
Seq ID No 48: Primer Eo7-23F; Seq ID No 49: Primer Eo7-23R;
Seq ID No 50: Primer Eo7-24F; Seq ID No 51: Primer Eo7-24R;
Seq ID No 52: Primer Eo7-25F; Seq ID No 53: Primer Eo7-25R;
Seq ID No 54: Primer Eo7-26F; Seq ID No 55: Primer Eo7-26R.

### DETAILED DESCRIPTION OF THE INVENTION

We have unexpectedly discovered the suitability for this purpose of a known Echo 7 type *Picornaviridae* enterovirus, isolated from a human intestine. The original nucleotide sequence, determined by a standard method, was found to be rather similar to that of Wallace type *Picornaviridae Enterovirus.*

Checking the oncolytic activity of isolated native enteroviruses in tissue of angiosarcoma demonstrated that neither individual viruses nor their combinations in a dose 3×10⁵ TCID₅₀/0.03 ml possessed substantial oncolytic activity with exception of ECHO 7 type that showed more promising activity (Table 1).

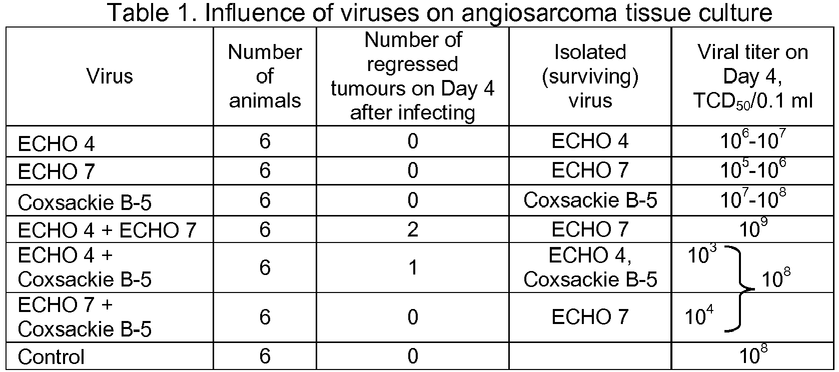

The instability of the genome of the RNA single strand viruses is a well-known fact; therefore, such viruses usually are not selected for constructing oncolytic viral agents.

The modification of the isolated native virus was realised in several consecutive steps.

The first step takes advantage of the high mutation potential of RNA viruses (on average one mutation on each replication) to develop a cytopathic mutant by replicating the virus in trypsinized monolayer of human embryonic fibroblast culture in the presence of calf serum.

Cells were incubated for 10 days, carrying out the passage each time the cells in culture had degenerated by 50%.

During testing of the selected virus in RD cell culture a pronounced cytopathic effect was already observed within 24 hours of infection. The titer of the developed virus, determined by last dilution method was TCID₅₀ =1 × 10⁻⁸. This strain was propagated, isolated and stored at -70 °C for further use in producing selective and genetically stable oncolytic strain.

The virus so obtained was modified, using a specially developed method comprising three steps.

### 1st modification step in a first tumour cell line

In the first modification step, the virus was propagated in tumour cell lines attenuated by anticancer agent. Human breast adenocarcinoma cell line (MCF-7) was used in this step.

A monolayer of these cells was treated with dacarbazine DTIC in sub toxic dose (20 µM). After treating with dacarbazine, the cells were transferred to fresh culture medium and contacted with the virus, and the propagation continued without adding serum. After 24 hours from contacting with the virus, the cells were removed and the virus was isolated from the media.

The virus was repeatedly propagated (passaged) in human embryonic fibroblast cell culture and again used for infecting the MCF-7 cell line. This procedure was repeated 10 times. Thus, this modification step comprises alternately propagating the virus in human breast adenocarcinoma cells and human embryonic fibroblast cells.

### 2nd modification step in a second tumour cell line

In the next modification step, the virus as described above, was contacted with gastric adenocarcinoma cell culture. A monolayer of these cells was treated with dacarbazine DTIC in sub toxic dose (20 µM).

The monolayer of these cells was infected by the virus, which was isolated after the modification in the first step, and the propagation continued in a culture medium without serum.

After 24 hours of contact with the virus, the cells were removed and the virus isolated from the media. The virus was repeatedly propagated (passaged) in human embryonic fibroblast cell culture, and thereafter again used for infecting the gastric adenocarcinoma cell line. This procedure was repeated 10 times. Thus, this second modification step comprises alternately propagating the virus in gastric adenocarcinoma cells and in human embryonic fibroblast cells.

In the first modification step and in the second modification step, the propagation procedure was always finished by propagating the virus last in the human embryonic fibroblast culture.

### 3rd modification step in human melanoma cells

The virus produced in the second step was used for infecting human tumours, obtained in surgery.

Melanoma cancer tissues were obtained in surgery from 23 patients previously treated by chemotherapy.

These tissues were infected with the modified virus and incubated at 37 °C in the absence of carbon dioxide. Before being used for infecting a new tissue material (fresh melanoma tissue from another patient), the modified virus was repeatedly propagated in human embryonic fibroblast culture to titer 7 lg TCID₅₀ /1 ml.

The modified virus was propagated in human embryonic fibroblast cell culture that was treated by 5 mM ribavirin 7 hours before infection and cultivated for 24 hours. The virus was isolated from the culture, and the procedure of propagating the virus in the fibroblast culture was repeated 10 times. Finally, the virus was isolated, purified and propagated in human embryonic fibroblast culture without addition of ribavirin.

The propagated virus was used for sequence determination. The genome of the modified virus differs from that of frozen unmodified native virus (Echo 7 type Wallace strain from NCBI database) by about 10%, the coat part by about 12%.

The virus modified by the described method was found to be surprisingly stable. Its genome changed by only 0.7% after continuous passaging for 12 months (propagation for 12 months in human embryonal lung culture MRC 5). Especially important is the fact that the modified virus (further on MV) is characterised by exceptionally high cytopathic effect on malignant cells and low cytotoxicity on normal cell lines as well as no toxicity *in vivo* in mice.

In experiments with cell lines MV was found to be cytotoxic for melanoma cell lines FM9, FM55, FM94 and SK-Mel26, gastric carcinoma cells, human oral squamous cell carcinoma SCC25 cells, human epithelial cell line derived from a lung carcinoma (A549), acute monocyte leukemia THP-1 cells, rabdomyosarcoma RD cells, human pancreatic adenocarcinoma HPAF-II cells, human breast adenocarcinoma cells (MCF-7) as well as on primary cell cultures of gastric adenocarcinoma GC1 and thyroid cancer line HA007.

In animal experiments, MV caused regression of murine sarcoma M-1, mice fibrosarcoma MX-17 as well as transplantable tumours - Moloney sarcoma (SM) and KRS-321 sarcoma.

In a clinical pilot study, a group of 46 melanoma stage I patients no progress of melanoma was observed for 50 months in 43 patients, treated with MV. In the control group, melanoma progressed for 10 of 31 patients undergoing standard therapy.

In a 50 months study of 44 stage II melanoma patients the progress of melanoma was stopped in 38 patients, compared to control group of 36 patients undergoing standard therapy, where melanoma did not progress in 15 patients, but did progress in 21 patient. No serious adverse effects were observed for patients treated with MV.

### INDUSTRIAL APPLICABILITY

We have developed a novel virus strain (MV) with original genome sequence, stable against genetic drift, possessing cytopathic activity against various types of tumours, characterized by low incidence of adverse effects and low toxicity that can be used with advantage in cancer virotherapy. Thus, we have unexpectedly solved the main obstacle in wider use of RNA viruses in medicine - obtained genetically stable strain that can be used in standardized continuous manufacturing of oncolytic viral preparation. The viral preparation can be used in anticancer therapy against a variety of tumour cells.

### EXAMPLES

The present invention is described in Examples in more detail. However, the invention is not construed as being limited to the examples.

### Virus

The virus modified according to the invention is ECHO-7 virus (Picornaviridae family, Enterovirus genus, ECHO (Enteric Cytopathic Human Orphan) type 7, group IV, positive-sense single stranded RNA virus). The native virus can be identified by genome sequence Seq Id No 2.

### Example 1. The isolation and characterization of the original virus strain

A known method for isolation (A.C. Rentz, J.E. Libbey, R.S. Fujinami, F.G. Whitby, and C.L. Byington. Investigation of Treatment Failure in Neonatal Echovirus 7 Infection. The Pediatric Infectious Disease Journal, Volume 25, Number 3, March 2006, 259) and propagation in BS-C-1 cell line (CCL 26; ATCC) was used (Libbey JE, McCright IJ, Tsunoda I, et al. Peripheral nerve protein, P0, as a potential receptor for Theiler's murine encephalomyelitis virus. J Neurovirol. 2001;7:97-104. Pevear DC, Tull TM, Seipel ME, et al. Activity of pleconaril against enteroviruses. Antimicrob Agents Chemother. 1999;43:2109-2115). Virus propagation and determination of titer was conducted in concordance with the published method (Zurbriggen A, Fujinam) RS. A neutralization-resistant Theiler's virus variant produces an altered disease pattern in the mouse central nervous system. J Virol. 1989;63:1505-1513).

### Example 2. Virus modification

In the first modification step, the virus was propagated in tumour cell lines attenuated by an anticancer agent. Initially, for propagation was used the human breast adenocarcinoma cell culture (MCF-7), cultivated in DME medium (Sigma-Aldrich) with 10% serum (Gibco) and antibiotics (100 IU/ml penicillin, 100 IU/ml streptomycin) at 37 °C under atmosphere, containing 5% CO₂ until developing of the monolayer.

The obtained monolayer of these cells was treated with dacarbazine DTIC in sub toxic dose (20 µM). After treating with dacarbazine cells were transferred to fresh culture medium without added serum, the cells contacted with virus and the propagation continued.

After 24 hours from contacting with the virus the cells were removed and virus isolated from the media. The virus was repeatedly propagated in human embryonal fibroblast cell culture and again used for infecting the MCF-7 cell line. This procedure was repeated 10 times.

In the next, second step, the virus as described above, was contacted with gastric adenocarcinoma cell culture. The cell culture for propagation was cultivated in DME medium (Sigma-Aldrich) with 10% serum (Gibco) and antibiotics (100 IU/ml penicillin, 100 IU/ml streptomycin) at 37 °C under atmosphere, containing 5% CO₂ until developing of the monolayer.

The obtained monolayer of these cells was treated with dacarbazine DTIC in sub toxic dose (20 µM). After treating with dacarbazine cells were transferred to fresh culture medium without added serum, the cells contacted with virus and the propagation continued.

After 24 hours from contacting with the virus the cells were removed and virus isolated from the media. The virus was repeatedly propagated in human embryonal fibroblast cell culture and again used for infecting the gastric adenocarcinoma cell line. This procedure was repeated 10 times.

In the third step, the virus produced in the second step was used for infecting human tumours, obtained in surgery. Melanoma cancer tissues were obtained in surgery from 23 patients previously treated by chemotherapy.

The tumour cells were separated from fat cells, necrotic tissue and blood, kept at 0 ⁰C for 24 hours, fragmented and as approximately 0.1 cm³ large tissue pieces immersed in Eagle medium (4 ml of medium for 10 mg of tissue), infected with the prepared virus and incubated in the absence of carbon dioxide at 37 ⁰C.

The medium was replaced by a fresh portion every day until the destruction of tumour, determined morphologically and visually by the oxidation level of medium.

The virus titer was determined every day in tumor tissue fee medium sample. The reproduction rate of virus was determined from the virus titer at the conclusion of an experiment in comparison with that on Day 0. Such modification of virus was performed in tissues obtained from 23 patients.

Before being used for infecting a new tissue material, the modified virus was each time repeatedly propagated in human embryonal fibroblast culture to titer 7 lg TCID ₅₀/1 ml.

The modified virus was propagated in human embryonal fibroblast cell culture that was treated by 5 mM ribavirin 7 hours before infection and cultivated for 24 hours. Virus was isolated from culture medium, and the procedure repeated 10 times.

Finally, the virus was isolated, purified and propagated in human embryonal fibroblast culture without addition of ribavirin.

The propagated virus sample was used for determination of genome sequence, anticancer activity and replicative stability by passaging it for 12 months in human embryonal fibroblast culture with repeated determination of genome sequence (Seq ID No 1).

### Example 3. Determination of virus genome sequence

The isolation, amplification and sequencing of the isolated, modified and cultivated virus genome were performed according to the known method [Chua BH, McMinn PC, Lam SK, Chua KB. Comparison of the complete nucleotide sequences of echovirus 7 strains UMMC and the prototype (Wallace) strain demonstrates significant genetic drift over time. J Gen Virol. 2001 Nov; 82(Pt 11): 2629-39].

For this purpose, 96 enteroviruses with complete genome sequence were selected from the NCBI Gene bank. The complete genome sequences for these viruses were downloaded and compared by Vector NTI program. Based on the results of comparing the most conservative regions of virus genomes were determined and 13 degenerated oligonucleotide pairs selected in these regions, covering the length of the potential enteroviruses genome. After the synthesis of the first 13 fragments, another 13 nucleotide pairs were produced. These oligonucleotide pairs were virus-specific and designed so as to produce overlaying fragments. After the building of the full genome sequence the virus genome was repeatedly sequenced with the virus-specific primers.

### Example 3.1. The genome sequence of the unmodified (native) virus

The sequence of the native virus was produced from 26 separate overlapping PCR fragments, synthesized from the primers listed in Table 2.

**Table 2. Primers used to sequence the complete genome of viruses.**

| **Primer** | **Sequence (5'- 3')** | **Length (bp)** | **Position** | **Target region** |
|---|---|---|---|---|
| **Eo7-1F** | **TTAAAACAGCCTGTGGGTTG** | **20** | **1-20** | **5' UTR** |
| **Eo7-1R** | **GAAACACGGACACCCAAAGTAG** | **22** | **545-566** | **5' UTR** |
| **Eo7-2F** | **CCATGGGACGCTTCAATACT** | **20** | **391-410** | **5' UTR** |
| **Eo7-2R** | **GCACCAGTCTTTTGTGTCGA** | **20** | **758-777** | **VP4** |
| **Eo7-3F** | **CGACTACTTTGGGTGTCCGTGTTTC** | **25** | **542-566** | **5' UTR** |
| **Eo7-3R** | **TCDGGRAAYTTCCACCACCACCC** | **23** | **1178-1200** | **VP2** |
| **Eo7-4F** | **CGACAGGGTGAGATCCCTAA** | **20** | **979-998** | **VP2** |
| **Eo7-4R** | **TTTCACCCTTCGTGAGGTTC** | **20** | **1381-1400** | **VP2** |
| **Eo7-5F** | **GCATCYAARTTYCAYCARGG** | **20** | **1289-1308** | **VP2** |
| **Eo7-5R** | **CACATKGGKGCAATSGTGAC** | **20** | **1676-1695** | **VP2** |
| **Eo7-6F** | **GTGGATCAACTTGCGCACTA** | **20** | **1513-1532** | **VP2** |
| **Eo7-6R** | **AAATTGTGGCATAGCCGAAG** | **20** | **1797-1816** | **VP3** |
| **Eo7-7F** | **GTCACSATTGCMCCMATGTG** | **20** | **1676-1695** | **VP2** |
| **Eo7-7R** | **CTTNATRCTYCCTGACCAGTGTG** | **23** | **2055-2077** | **VP3** |
| **Eo7-8F** | **AAGCATGGACGCATATCACA** | **20** | **1921-1940** | **VP3** |
| **Eo7-8R** | **GATATGGGTTCCCACATTGC** | **20** | **2174-2194** | **VP3** |
| **Eo7-9F** | **CACACTGGTCAGGRAGYATNAAG** | **23** | **2055-2077** | **VP3** |
| **Eo7-10F** | **CAAGTGTGTCGTCCTGTGCT** | **20** | **2350-2369** | **VP3** |
| **Eo7-9R** | **CCTATTGGCGCTGTCTTGAT** | **20** | **2694-2713** | **VP1** |
| **Eo7-11F** | **ACCAAAGATCAAGACAGCGC** | **20** | **2687-2706** | **VP1** |
| **Eo7-11R** | **TTGGCACCCACACTCTGATA** | **20** | **3178-3197** | **VP1** |
| **Eo7-12F** | **ACCAGTCCGGTGCTGTTTAC** | **20** | **3336-3355** | **VP1-2A** |
| **Eo7-12R** | **TCCCAYACACARTTYTGCCAGTC** | **23** | **3401-3423** | **2A** |
| **Eo7-13F** | **CARAAYTGTGTGTGGGAAGACTA** | **23** | **3407-3429** | **2A** |
| **Eo7-13R** | **CCCTGYTCCATKGCTTCATCYTCYARC** | **27** | **3748-3774** | **2A-2B** |
| **Eo7-14F** | **TTACCCAGTCACCTTCGAGG** | **20** | **3535-3554** | **2A** |
| **Eo7-14R** | **TGTTTTTCCTTCACTTCCGG** | **20** | **4181-4200** | **2C** |
| **Eo7-15F** | **GTTRGARGATGATGCNATGGARCARGG** | **27** | **3748-3774** | **2A-2B** |
| **Eo7-15R** | **TCAATACGGYRTTTGSWCTTGAA** | **23** | **4409-4431** | **2C** |
| **Eo7-16F** | **CCTYTRTAYGCVGCYGARGC** | **20** | **4343-4362** | **2C** |
| **Eo7-17F** | **TTCAAGWSCAAAYRCCGTATTGA** | **23** | **4409-4431** | **2C** |
| **Eo7-16R** | **AAYTGAATGGCCTTHCCACACAC** | **23** | **4922-4944** | **2C** |
| **Eo7-18F** | **CTDGTGTGTGGRAAGGCYATNCA** | **23** | **4919-4941** | **2C** |
| **Eo7-18R** | **TATGCTCCYTGRAARCCTGCAAA** | **23** | **5309-5330** | **3A-3B** |
| **Eo7-19F** | **CAAGCCCTAACCACGTTTGT** | **20** | **5252-5271** | **3A** |
| **Eo7-19R** | **ACCCGTAGTCAGTCACCTGG** | **20** | **5740-5759** | **3C** |
| **Eo7-20F** | **TTTGCAGGMTTYCARGGWGCATA** | **23** | **5309-5330** | **3A-3B** |
| **Eo7-20R** | **GCYCTWGTGGGRAAGTTRTACAT** | **23** | **5723-5745** | **3C** |
| **Eo7-21F** | **GTGTTGGATGCCAAGGAACT** | **20** | **5555-5574** | **3C** |
| **Eo7-21R** | **ATGGGCTCCGATCTGATGTC** | **20** | **6203-6222** | **3D** |
| **Eo7-22F** | **TTCCCCACWAGRGCAGGCCARTGYGG** | **26** | **5907-5832** | **3C** |
| **Eo7-22R** | **CTCCAAAABASRTCYGGGTCRCA** | **23** | **6572-6594** | **3D** |
| **Eo7-23F** | **TGAAGGAATGCATGGACAAA** | **20** | **6360-6379** | **3D** |
| **Eo7-23R** | **ATGGGTATTGCTCATCTGCC** | **20** | **7078-7097** | **3D** |
| **Eo7-24F** | **TGYGACCCRGAYSTVTTTTGGAG** | **23** | **6572-6594** | **3D** |
| **Eo7-24R** | **TCRTGDATDTCYTTCATGGGCA** | **22** | **7116-7137** | **3D** |
| **Eo7-25F** | **CCTGGACGAATGTGACCTTT** | **20** | **7041-7060** | **3D** |
| **Eo7-25R** | **CCCTACCGCACTTTTATCCA** | **20** | **7384-7403** | **3'UTR** |
| **Eo7-26F** | **ATCCAYGARTCHATYAGRTGGAC** | **23** | **7130-7152** | **3D** |
| **Eo7-26R** | **CCGCACCGAATGCGGAGAATTTAC** | **24** | **7404-7427** | **3'UTR** |

| | | | | |
|---|---|---|---|---|
| **UTR - untranslated region.** | | | | |

The 5'-terminal and the 3'-terminal sequences were obtained, using 5'-RACE and 3'-RACE methods, correspondingly.

As a result, the full genome sequence of the unmodified virus was found to consist of 7434 nucleotides, excluding the poly A sequence (Seq ID No 2). The untranslatable 5'-terminal (5'NTR) contains 742 nucleotides, followed by coding part starting with start codon (AUG) at position 743, containing codons for 2196 amino acids and ending with stop codon (UAA) at position 7331 (Seq ID No 2). The untranslatable 3'-terminal (3'NTR) of this strain contains 100 nucleotides, followed by poly A sequence.

### Example 3.2. The sequence of the modified virus (MV)

The sequence of the starting virus was produced from 26 separate overlapping PCR fragments, synthesized using the primers listed in Table 2.

The 5'-terminal and the 3'-terminal sequences were obtained, using 5'-RACE and 3'-RACE methods, correspondingly.

As a result, the full genome sequence of the modified virus was found to consist of 7427 nucleotides, excluding the poly A sequence (Seq ID No 1).

The untranslatable 5'-terminal (5'NTR) of this strain contains 742 nucleotides, followed by the coding sequence. The coding part that contains information about the virus polyprotein, begins with the start codon (AUG) at position 743, contains codons for 2194 amino acids and ends with stop codon (UAA) at position 7325 (Seq ID No 1). The untranslatable 3'-terminal (3'NTR) of this strain contains 100 nucleotides, followed by poly A sequence.

### Example 3.3. The genome sequence of the modified virus after propagation for 12 months

The sequence of the modified virus was produced from 26 separate overlapping PCR fragments, synthesized the primers listed in Table 2.

The 5'-terminal and the 3'-terminal sequences of this strain were obtained, using 5'-RACE and 3'-RACE methods, correspondingly.

As a result, the full genome sequence of the modified virus was found to consist of 7427 nucleotides, excluding the poly a sequence (Seq ID No 3). The untranslatable 5'-terminal (5'NTR) contains 742 nucleotides, followed by coding part, starting with start codon (AUG) at position 743, containing codons for 2194 amino acids and ending with stop codon (UAA) at position 7325 (Seq ID No 3). The untranslatable 3'-terminal (3'NTR) of this strain contains 100 nucleotides, followed by poly A sequence.

### Example 3.4. Comparison of genomes of modified virus (MV) and native strain

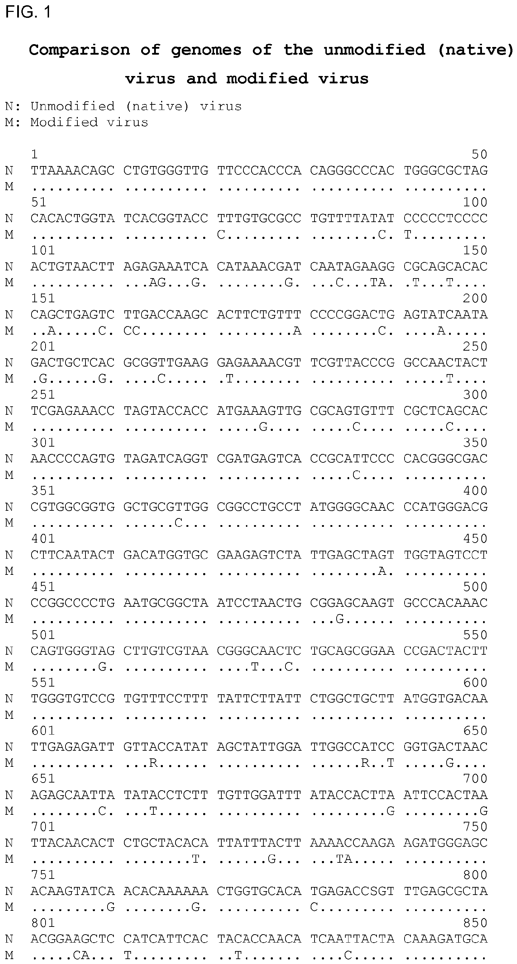

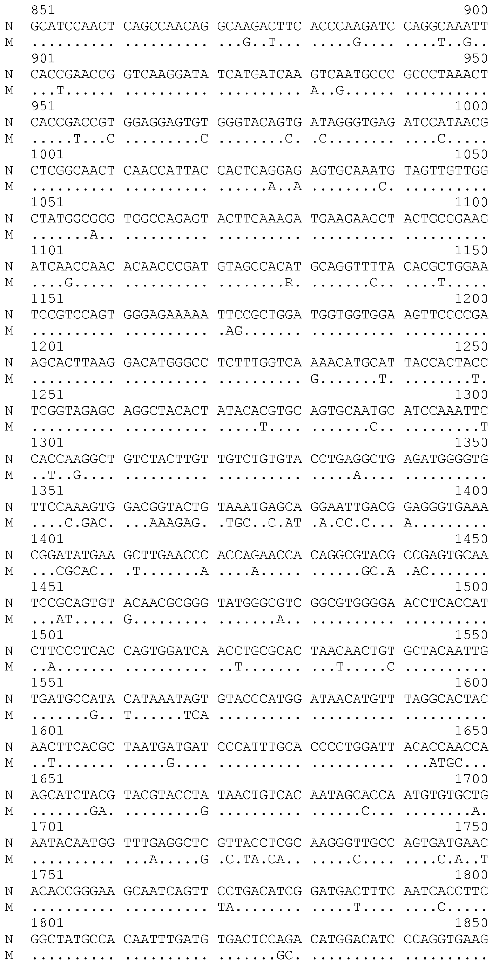

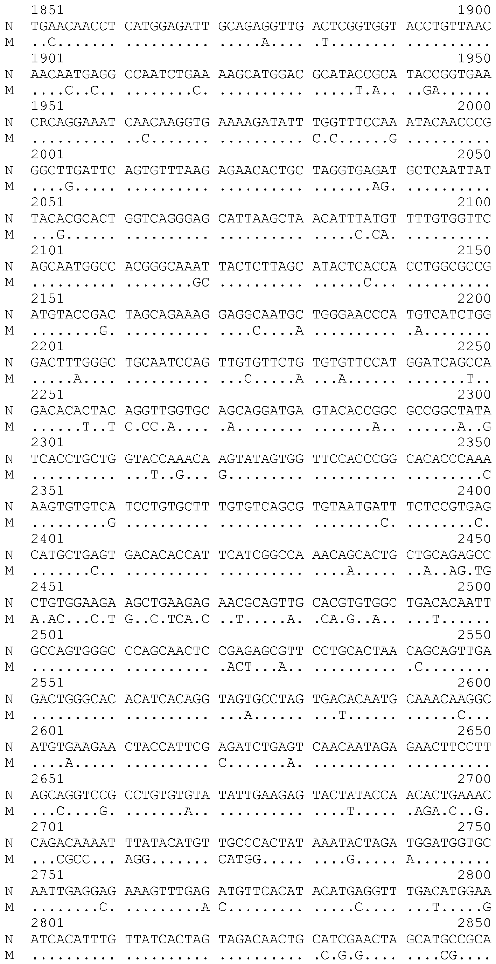

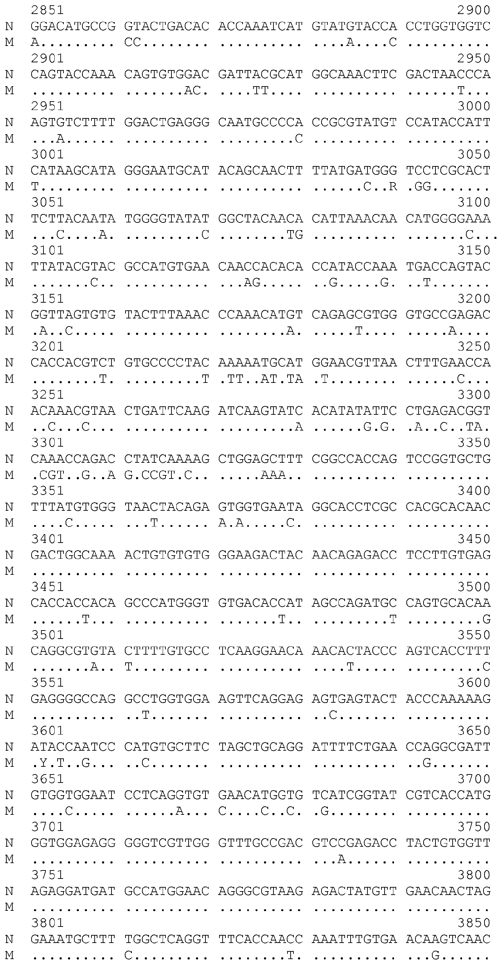

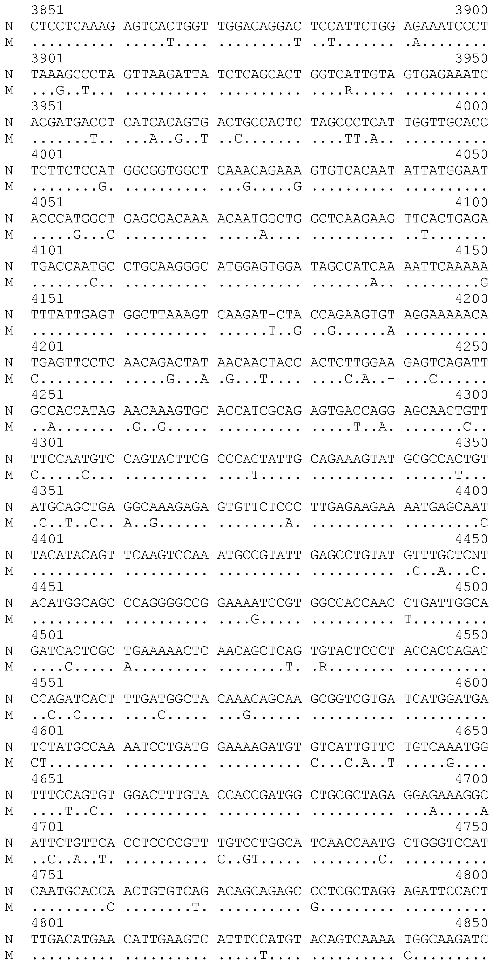

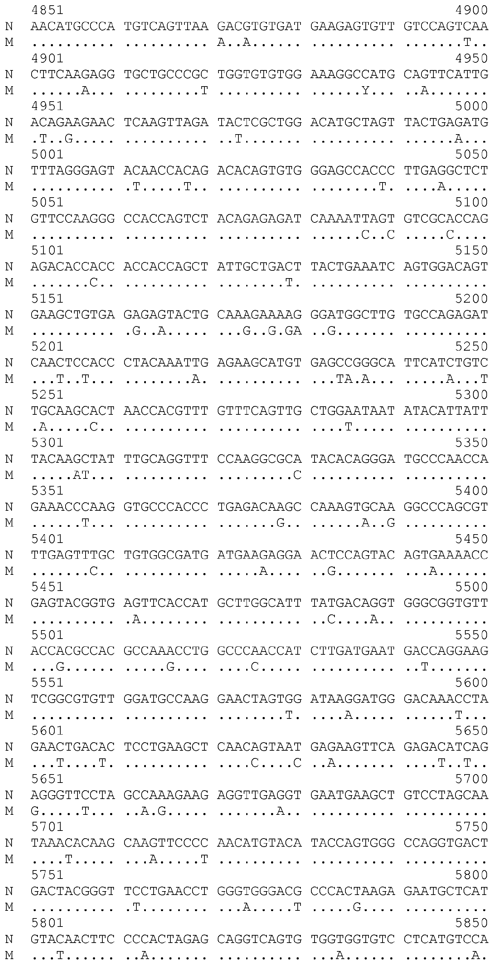

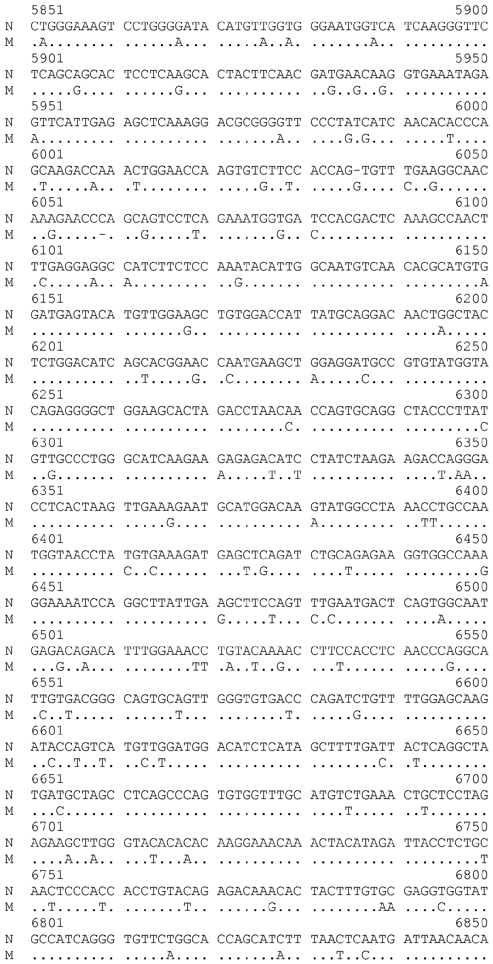

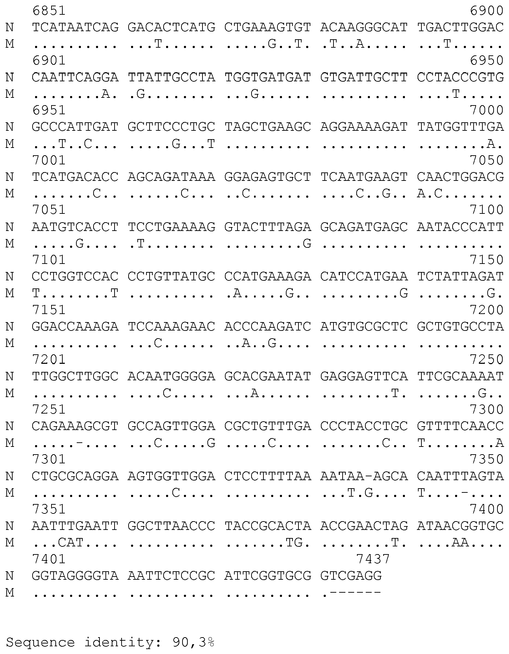

Comparison of genomes of modified virus (MV) and starting strain is provided in Fig. 1.

The difference in nucleotide sequence, calculated by programme Vector NTI is substantial, 10% for the complete genome and 12% for the part coding the virus coat proteins. The amino acid sequences for the modified and starting strains are listed inFig. 2.

### Example 3.5. The genome sequence of the modified virus after propagation for 12 months

The changes in the sequence of modified virus (MV) genome after continuous passaging for 12 months did not exceed 0.7% of the initial sequence.

All found changes were one nucleotide replacements, partially the mute mutations (without change of amino acid). If the amino acid was changed, its position was in the genome polymorphic part, evidently without relevant influence on virus activity.

### Example 4. Virus passaging

Virus MV was passaged by known methods and propagated for 12 months in human embryonal lung culture MRC 5 (Instituto Zooprofilattico Sperimentale della Lombardia e dell Emilia, Brescia - Laboratorio Centro Substrati Cellulari, Catalogue No. BS CL 68 (origin: American Type Culture centre Collection, Rockville, Md, USA), free of bacteria, viruses, fungi or mycoplasmas, and later stored frozen at -70 ⁰C.

### Example 5. Determination of anti-cancer activity of the modified virus (MV)

In experiments with cell lines, MV was found to be cytotoxic for melanoma cell lines FM9, FM55, FM94 and SK-Mel26, gastric carcinoma cells, human oral squamous cell carcinoma SCC25 cells, human epithelial cell line derived from a lung carcinoma (A549), acute monocyte leukemia THP-1 cells, rabdomiosarcoma RD cells, human pancreatic adenocarcinoma HPAF-II cells, human breast adenocarcinoma cells (MCF-7) as well as on primary cell cultures of gastric adenocarcinoma GC1 and thyroid cancer line HA007. Thus, for example, MV injections for 3 days caused reducing of sarcoma M-1 mass in 55% (in 11 of 22) of animals, compared with 6% (in 1 of 18) spontaneous regression in the control group.

Transplanting sarcoma KRS-321 on Day 5 after the injecting MV in a dose 15×10⁶ TCID₅₀ on Wistar rats in 44% of animals (11/25) the regression of tumour was observed, while in the control group there were no cases of regression.

Testing the anti-cancer activity of the virus sample after the 12 months passaging on the same cancer cell lines and transplanted tumours no statistically significant difference from the original MV was observed.

Neither MV nor the virus passaged for 12 months caused any toxic reactions in intact mice.

### Example 6. Anti-cancer activity of modified virus in treating patients

Treating of melanoma patients by the modified virus (MV) was conducted according to the following scheme: therapy was commenced 2-3 weeks after the excision of the tumour by intramuscular administration of 2 ml of solution with titer 2×10⁶ TCID₅₀/ml - 2×10⁸ TCID₅₀/ml for 3 days consecutively with supporting injections at monthly intervals according to the same 3 day schedule. After the fourth month, the virus preparation was administered once monthly for the next 8 months. In the next 2 years the supporting therapy was continued with the same dose, gradually increasing the interval between administrations to 6, 8 and 12 weeks.

In a clinical pilot study, a group of 46 melanoma stage I patients no progress of melanoma was observed for 50 months in 43 patients, treated with MV. In the control group, melanoma progressed for 10 of 31 patients undergoing standard therapy.

In a 50 months study of 44 stage II melanoma patients the progress of melanoma was stopped in 38 patients, compared to control group of 36 patients undergoing standard therapy, where melanoma did not progress in 15 patients, but did progress in 21 patients.

The efficiency of treatment is characterized by the following examples:
Case 1. Female, age 76 , *Melanoma cutis dorsi*
   Op. 11.09.2009. *Excisio tu cutis dorsi*
   pT4b N0 M0
   SN biopsy was not performed
   *Ex consilio:* follow-up
   Op. 07.04.2010. *LAE axillaris sin.*
   *Mts Iln axillaris sin*
   *Ex consilio: Roferon*
   *Roferon* 6 mil 3x per week from 24.06.2010 till 30.08.2010.
   The treatment was discontinued due to the side effects.
   From October 2010 the therapy with virus preparation in 2 ml dose with titer 2×10⁶ *TCID₅₀*/*ml* - 2×10⁸ *TCID₅₀*/*ml* was commenced. The treatment was well tolerated, and no progression of the disease was documented until 01.02.2012.
Case 2. Female, age 42 , *Melanoma cutis dorsi*
   Op. 25.05.2008. *Excisio tu cutis dorsi*
   pT4a N0 M0, *Clark V, Breslow9* mm
   SN biopsy was not performed
   Virus preparation (2 ml with titer *2*×*10⁶ TCID₅₀*/*ml* - *2*×*10⁸ TCID₅₀*/*ml was administered* from 27.06.2008 till 27.06.2011.
   21.01.2011. US examination: recurrence in the scar
   Op. 02.02.2011. *Excisio.* Histological examination: granuloma.
   Virus preparation (2 ml with titer *2*×*10⁶ TCID₅₀*/*ml* - *2*×*10⁸ TCID₅₀*/*ml was continued* till 27.06.2011.
   During the observation period (till December 2011) no evidence of the disease progression was documented.
Case 3. Female, age 57 , *Melanoma cutis dorsi*
   Op.19.08.2007. *Excisio tu cutis dorsi*
   P T3b N0 M0
   SN biopsy was not performed
   Recommendations: follow-up
   Op. 10.12.2009. *LAE colli dx.* Histological examination: *mts I*/*n colli dx* Progression of the disease - US examination on 22.02.2010: *mts Iln colli* 22.02.2010. *Ex consilio:* no surgery was recommended due to bulky disease Virus preparation (2 ml with titer *2*×*10⁶ TCID₅₀*/*ml* - *2*×*10⁸ TCID₅₀*/*ml was administered* from 22.02.2010 and still is in progress.
   Last visit at clinic on 22.11.2011 - the disease has stabilized.
Case 4. Female, age 58 , *Melanoma cutis dorsi*
   Op. April 2004. *Excisio tu cutis dorsi, LAE axillaris sin.*
   pT4b, N2c, M0 (*Breslow* 15 mm)
   *Reexcisio* January 2006, September 2006 (local recurrence)
   Therapy with IFN from October 2006 till May 2007.
   *Reexcisio cum dermoplasticum* February 2007, May 2007, September 2007. Virus preparation (2 ml with titer *2*×*10⁶ TCID₅₀*/*ml* - *2*×*10⁸ TCID₅₀*/*ml was administrated from February 2008 till April* 2011.
   Visceral metastasis February 2011.
   *Exitus letalis* October 2011.

### Dose form and administration

The viral preparation for therapeutic treatment can be in the form of injectable aqueous solution containing the modified virus having the stable genome sequence as explained above, for example in the titer of 2×10⁶ TCID₅₀/ml - *2*×*10⁸* TCID₅₀/ml. The solution carrying the virus can be any physiologically acceptable sterile solution, especially sodium chloride solution. The preparation is stored and transported in frozen condition and defrozen at room temperature before the use. The preparation can be in vials of other container units in volumes that correspond a single dose injected at a time to the patient.

The preparation can be administered by injecting it intramuscularly (i.m.) to the patient after the excision of the tumour in question, when the wound has healed. The dosage can be 2 ml of the above-mentioned solution at a time The intramuscular administration by injection is repeated according to the planned therapy schedule.

## Claims

1. Modified enterovirus of ECHO 7 type **characterized by** stable genome sequence that has at least 85%, preferably at least 95%, still more preferably at least 99% of sequence identical to Seq ID No 1.

2. Modified enterovirus of ECHO 7 type, **characterized by** a stable genome sequence of Seq ID No 1.

3. Modified enterovirus of claim 1 or 2, wherein the changes in the sequence of the genome of the modified enterovirus are not larger than 1.0%, preferably not larger than 0.7% after continuous propagation of the modified enterovirus in cell cultures for 12 months.

4. Method for manufacturing a modified enterovirus of ECHO 7 type by modification of native ECHO 7 virus, identified by genome sequence Seq ID No 2, wherein the modification comprises conducting the virus adaptation in cancer cells, attenuated by an anti-cancer agent, subsequently passaging the modified virus in human embryonal fibroblast culture, propagating the modified virus in human melanoma cells, and subsequently passaging the modified virus in human embryonal fibroblast culture, optionally treated by ribavirin, and isolation and purification of the virus.

5. Method of claim 4, wherein the procedure of propagating the virus in cancer cells and subsequently passaging the modified virus in human embryonal fibroblast culture is repeated several times.

6. Method of claim 4 or 5, wherein the procedure of propagating the modified virus in human melanoma cells, and subsequently passaging the modified virus in human embryonal fibroblast culture is repeated several times.

7. Method of claim 4, 5 or 6, wherein the virus adaptation is conducted in cancer cells of at least two different cancers, such as human breast adenocarcinoma cells and gastric adenocarcinoma cells.

8. Method of any of claims 4-7, wherein the modification gives a modified enterovirus of ECHO 7 type, which is **characterized by** stable genome sequence that has at least 85%, preferably at least 95%, still more preferably at least 99% of sequence identical to Seq ID No 1.

9. Method of any of claims 4-7, wherein the modification gives a modified enterovirus of ECHO 7 type, which is **characterized by** a stable genome sequence of Seq ID No 1.

10. Method of claim 8 or 9, wherein in the modified enterovirus having the stable genome sequence, the changes in the sequence of the genome are not larger than 1.0%, preferably not larger than 0.7% after continuous propagation of the modified enterovirus in cell cultures for 12 months.

11. Method of claim 10, wherein the changes are one nucleotide replacements, which consist partly of mute mutations without change of corresponding amino acid.

12. The virus of Claim 1, 2 or 3 for use in treating oncological diseases.

13. The virus for use of Claim 12, wherein the oncological disease is selected from the group consisting of: melanoma, gastric cancer, intestinal cancer, human breast cancer, prostate cancer, pancreatic cancer, lung cancer, kidney cancer, bladder cancer, lymphosarcoma, uterine cancer, angiosarcoma, rhabdomyosarcoma.

14. The virus for use of Claim 12 wherein the oncological disease is melanoma.

## Patentansprüche

1. Modifiziertes Enterovirus vom Typ ECHO 7, **gekennzeichnet durch** eine stabile Genomsequenz, die mindestens 85%, vorzugsweise mindestens 95%, noch mehr bevorzugt mindestens 99% sequenzidentisch mit Seq ID Nr. 1 ist.

2. Modifiziertes Enterovirus vom Typ ECHO 7, **gekennzeichnet durch** eine stabile Genomsequenz Seq ID Nr. 1.

3. Modifiziertes Enterovirus nach Anspruch 1 oder 2, worin die Änderungen in der Genomsequenz des modifizierten Enterovirus nach kontinuierlicher Vermehrung des modifizierten Enterovirus in Zellkulturen über 12 Monate nicht größer als 1,0%, vorzugsweise nicht größer als 0,7% sind.

4. Verfahren zur Herstellung eines modifizierten Enterovirus vom Typ ECHO 7 durch Modifikation eines nativen ECHO 7 Virus, identifiziert durch Genomsequenz Seq ID Nr. 2, worin die Modifikation die Durchführung der Virusadaption in durch ein Anti-Krebsmittel abgeschwächten Krebszellen umfasst, anschließendes Passagieren des modifizierten Virus in humaner embryonaler Fibroblastenkultur, Vermehren des modifizierten Virus in humanen Melanomzellen und anschließendes Passagieren des modifizierten Virus in humaner embryonaler Fibroblastenkultur, gegebenenfalls behandelt mit Ribavirin und Isolierung und Reinigung des Virus.

5. Verfahren nach Anspruch 4, worin die Maßnahme zum Vermehren des Virus in Krebszellen und anschließendes Passagieren des modifizierten Virus in humaner embryonaler Fibroblastenkultur mehrfach wiederholt werden.

6. Verfahren nach Anspruch 4 oder 5, worin die Maßnahme zum Vermehren des modifizierten Virus in humanen Melanomzellen und anschließendes Passagieren des modifizierten Virus in humaner embryonaler Fibroblastenkultur mehrfach wiederholt werden.

7. Verfahren nach Anspruch 4, 5 oder 6, worin die Adaption des Virus in den Krebszellen von mindestens zwei verschiedenen Krebsarten durchgeführt wird, wie z.B. humanen Mamma-Adenokarzinom- und gastrischen Adenokarzinomzellen.

8. Verfahren nach einem der Ansprüche 4-7, worin die Modifikation ein modifiziertes Enterovirus vom Typ ECHO 7 ergibt, welches durch eine stabile Genomsequenz gekennzeichnet ist, die mindestens 85%, vorzugsweise mindestens 95%, noch mehr bevorzugt mindestens 99% sequenzidentisch mit Seq ID Nr. 1 ist.

9. Verfahren nach einem der Ansprüche 4-7, worin die Modifikation ein modifiziertes Enterovirus vom Typ ECHO 7 ergibt, welches durch die stabile Genomsequenz Seq ID Nr. 1 gekennzeichnet ist.

10. Verfahren nach Anspruch 8 oder 9, worin die Änderungen in der Genomsequenz des modifizierten Enterovirus mit stabiler Genomsequenz nach kontinuierlicher Vermehrung des modifizierten Enterovirus in Zellkulturen über 12 Monate nicht größer als 1,0%, vorzugsweise nicht größer als 0,7% sind.

11. Verfahren nach Anspruch 10, worin die Änderungen Ein-Nukleotid-Austausche sind, die teilweise aus stummen Mutationen ohne Änderung der entsprechenden Aminosäure bestehen.

12. Das Virus nach Anspruch 1, 2 oder 3 zur Verwendung in der Behandlung von onkologischen Erkrankungen.

13. Das Virus zur Verwendung nach Anspruch 12, worin die onkologische Erkrankung aus der Gruppe ausgewählt ist, bestehend aus: Melanom, Magenkrebs, Darmkrebs, humanem Brustkrebs, Prostatakrebs, Bauchspeicheldrüsenkrebs, Lungenkrebs, Nierenkrebs, Blasenkrebs, Lymphosarkom , Gebärmutterkrebs, Angiosarkom, Rhabdomyosarkom.

14. Das Virus zur Verwendung nach Anspruch 12, worin die onkologische Erkrankung ein Melanom ist.

## Revendications

1. Entérovirus de type ECHO 7 modifié **caractérisé par** une séquence génomique stable qui est identique à au moins 85 %, préférentiellement au moins 95 %, encore plus préférentiellement au moins 99 % à SEQ ID NO: 1.

2. Entérovirus de type ECHO 7 modifié, **caractérisé par** une séquence génomique stable de SEQ ID NO: 1.

3. Entérovirus modifié selon la revendication 1 ou 2, dans lequel les changements dans la séquence du génome de l'entérovirus modifié ne sont pas supérieurs à 1,0 %, de préférence pas supérieurs à 0,7 % après propagation continue de l'entérovirus modifié dans des cultures cellulaires pendant 12 mois.

4. Procédé de fabrication d'un entérovirus de type ECHO 7 modifié par modification du virus ECHO 7 natif, identifié par la séquence génomique SEQ ID NO: 2, dans lequel la modification comprend la conduction de l'adaptation du virus dans des cellules cancéreuses, atténuées par un agent anticancéreux, ensuite le passage du virus modifié dans une culture de fibroblastes embryonnaires humains, la propagation du virus modifié dans des cellules de mélanome humain, et ensuite le passage du virus modifié dans une culture de fibroblastes embryonnaires humains, optionnellement traitée par la ribavirine, et l'isolement et la purification du virus.

5. Procédé selon la revendication 4, dans lequel la procédure de propagation du virus dans des cellules cancéreuses et ensuite du passage du virus modifié dans une culture de fibroblastes embryonnaires humains est répétée plusieurs fois.

6. Procédé selon la revendication 4 ou 5, dans lequel la procédure de propagation du virus modifié dans des cellules de mélanome humain, et ensuite du passage du virus modifié dans une culture de fibroblastes embryonnaires humains est répétée plusieurs fois.

7. Procédé selon la revendication 4,
5 ou 6, dans lequel l'adaptation du virus est conduite dans des cellules cancéreuses d'au moins deux cancers différents, telles que des cellules d'adénocarcinome du sein humain et des cellules d'adénocarcinome gastrique.

8. Procédé selon l'une quelconque des revendications 4 à 7, dans lequel la modification donne un entérovirus de type ECHO 7 modifié, qui est **caractérisé par** une séquence génomique stable qui est identique à au moins 85 %, préférentiellement au moins 95 %, encore plus préférentiellement au moins 99 % à SEQ ID NO: 1.

9. Procédé selon l'une quelconque des revendications 4 à 7, dans lequel la modification donne un entérovirus de type ECHO 7 modifié, qui est **caractérisé par** une séquence génomique stable de SEQ ID NO: 1.

10. Procédé selon la revendication 8 ou 9, dans lequel dans l'entérovirus modifié ayant la séquence génomique stable, les changements dans la séquence du génome ne sont pas supérieurs à 1,0 %, de préférence pas supérieurs à 0,7 % après propagation continue de l'entérovirus modifié dans des cultures cellulaires pendant 12 mois.

11. Procédé selon la revendication 10, dans lequel les changements sont des remplacements d'un nucléotide, qui consistent en partie en des mutations silencieuses sans changement de l'acide aminé correspondant.

12. Virus selon la revendication 1, 2 ou 3 pour une utilisation dans le traitement de maladies oncologiques.

13. Virus pour une utilisation selon la revendication 12, dans lequel la maladie oncologique est choisie dans le groupe constitué de: mélanome, cancer gastrique, cancer intestinal, cancer du sein humain, cancer de la prostate, cancer pancréatique, cancer du poumon, cancer du rein, cancer de la vessie, lymphosarcome, cancer de l'utérus, angiosarcome, rhabdomyosarcome.

14. Virus pour une utilisation selon
la revendication 12 dans lequel la maladie oncologique est un mélanome.
